# NEUE EUROPÄISCHE PATENTSCHRIFT

(11) **EP 1 395 297 B2**
(45) Veröffentlichungstag und Bekanntmachung des Hinweises auf die Entscheidung über den Einspruch: **30.06.2010**
(45) Hinweis auf die Patenterteilung: 26.04.2006
(21) Anmeldenummer: 02740703.0
(22) Anmeldetag: 11.06.2002
(51) Int. Cl.: A61L 27/04, A61L 27/58

(54) **MEDIZINISCHES IMPLANTAT FÜR DEN MENSCHLICHEN ODER TIERISCHEN KÖRPER**
MEDICAL IMPLANT FOR THE HUMAN OR ANIMAL BODY
IMPLANT MEDICAL POUR LE CORPS HUMAIN OU ANIMAL

(30) Priorität: 11.06.2001 DE 10128100
(43) Veröffentlichungstag der Anmeldung: 10.03.2004
(73) Patentinhaber: Biotronik VI Patent AG, 6341 Baar (CH)
(72) Erfinder: MEYER-LINDENBERG, Andrea, 31275 Lehrte (DE); WINDHAGEN, Henning, 30159 Hannover (DE); WITTE, Frank, 30625 Hannover (DE); NIEMEYER, Matthias, 30163 Hannover (DE); KAESE, Volker, 30161 Hannover (DE)
(74) Vertreter: Lindner-Vogt, Karin L.
(86) Internationale Anmeldenummer: PCT/EP2002/006375
(87) Internationale Veröffentlichungsnummer: WO 2002/100452

(56) Entgegenhaltungen:
- EP-A- 0 719 870
- EP-A- 0 765 947
- WO-A-99/03515
- DE-A- 2 010 841
- DE-A- 19 731 021
- US-A- 4 126 242

## Beschreibung

Die Erfindung betrifft ein medizinisches Implantat für den menschlichen oder tierischen Körper, das zumindest teilweise aus einer Magnesiumlegierung besteht.

Medizinische Implantate der genannten Art sind in verschiedenen Formen bekannt. Es kann sich dabei um Befestigungselemente für einen Knochen, beispielsweise Platten, Schrauben oder Nägel, um chirurgisches Nahtmaterial, um chirurgische Netze oder Folien oder auch um Prothesen oder Prothesenteile handeln. Üblicherweise bestehen zur Zeit verwendete Implantate aus korrosionsbeständigem Material wie Edelstahl oder Titan. Damit ist jedoch der Nachteil verbunden, daß die Implantate im Körper nicht degradiert werden und deshalb operativ entfernt werden müssen, wenn sie aus medizinischen Gründen nicht mehr notwendig sind, da es ansonsten zu Gegenreaktionen des Körpers kommen kann. Alternativ sind auch degradierbare Implantate aus Polymeren bekannt. Sie besitzen jedoch eine relativ geringe Festigkeit und Duktilität.

Bereits seit Beginn des 20. Jahrhunderts ist es bekannt, daß Implantate aus Magnesium oder Magnesiumlegierungen gewisse Vorteile mit sich bringen, da Magnesium leicht degradierbar ist. In dem Aufsatz "Magnesium Screw and Nail Transfixion in Fractures" von Earl D. McBride aus "Southern Medical Journal, 1938, Vol. 31, Nr. 5, S. 508 ff. ist die Verwendung von Schrauben, Bolzen und Dübeln aus Magnesium bzw. Magnesiumlegierungen beschrieben. In der DE 197 31 021 A1 wird diese Idee wieder aufgegriffen, ohne jedoch auf die bekannten degradierbaren Implantate aus Magnesium oder Magnesiumlegierungen einzugehen. Die im Zusammenhang mit der Knochenchirurgie beschriebenen Magnesiumlegierungen weisen jedoch den Nachteil auf, daß sie eine relativ große Gasmenge pro Zeiteinheit, insbesondere an Wasserstoff produzieren. Dadurch besteht die Gefahr, daß in dem mit einem entsprechenden Implantat versehenen Körper Gaskavernen entstehen, die für den Heilungsprozeß hinderlich sind, da sie insbesondere das Gewebe und die Gewebeschichten voneinander trennen. Darüber hinaus zeigen die bekannten Magnesiumlegierungen einen ungleichförmigen Korrosionsangriff, der einen zuverlässigen Halt während des notwendigen Heilungszeitraums nicht gewährleistet.

Auch chirurgisches Nahtmaterial aus Magnesium oder Magnesiumlegierungen ist seit langem bekannt, wie sich beispielsweise aus DE-PS 630 061, DE-PS 676 059, DE-PS 665 836 und DE-PS 688 616 ergibt. Mit einem derartigen Nahtmaterial sind ebenfalls die oben genannten Nachteile hinsichtlich der Gasentwicklung und des ungleichmäßigen Korrosionsangriff gegeben.

Es ist ferner bekannt, Magnesium oder eine Magnesiumlegierung auf Implantate insbesondere aus Edelstahl aufzudampfen, da diese Stoffe zu einer rascheren Wiederherstellung des Knochens beitragen. Auch aus entsprechendem Material bestehende Prothesen oder Prothesenteile wurden bereits verwendet. Um das Knochenwachstum zu fördern, können den Legierungen Calcium und Cadmium zugeschlagen sein. Neben den bereits oben genannten Nachteilen ist insbesondere die Verwendung von Cadmium (Cd) problematisch, da es sich hierbei um ein toxisches Metall handelt, das nicht in den Körper gelangen sollte.

Der Erfindung liegt die Aufgabe zugrunde, ein medizinisches Implantat für den menschlichen oder tierischen Körper vorzusehen, das die vorgenannten Nachteile vermeidet und mit keinen oder nur geringen Nebenwirkungen im Körper degradierbar ist.

Diese Aufgabe wird erfindungsgemäß bei einem medizinischen Implantat der genannten Art mit den Merkmalen des Anspruchs 1 gelöst. Der in der Magnesiumlegierung enthaltene Seltenerdmetallanteil fängt den bei der Korrosion des Magnesiums entstehenden Wasserstoff ab. Durch die Beimischung der Seltenerdmetalle zur Magnesiumlegierung wird eine Kornfeinung erreicht, wodurch sich ein langsamer, kontinuierlicher und gut vorhersehbarer Korrosionsverlauf des entsprechenden Implantates im Körper einstellt. Auf diese Weise sind eine übermäßige Gasentwicklung und die Gefahr der Bildung von Gaskavernen bei der Degradierung des Implantates zuverlässig vermieden. Durch die lithiumabhängige Deckschichtkomponentenvermehrung wird ein sehr guter Korrosionsschutz der Magnesiumlegierung erreicht.

Das Zulegieren von Seltenerdmetallen zu Magnesiumbasislegierungen verbessert darüber hinaus deren mechanische Materialeigenschaften. Die erfindungsgemäße degradierbare Legierung zeichnet sich durch eine erhöhte Duktilität und eine gesteigerte Festigkeit bei gleichzeitig gutem Korrosionswiderstand im Vergleich zu den bekannten degradierbaren Magnesiumlegierungen für Implantate aus.

Erfindungsgemäß finden als Seltenerdmetalle Cer und/oder Neodym und/oder Praseodym oder ein anderes Element der Ordnungszahlen 57 bis 71 des Periodensystems Verwendung. Dabei dürfte Cer zu bevorzugen sein, da es in natürlicher Weise im Körper und insbesondere im Knochen vorkommt.

Gemäß der Erfindung ist vorgesehen, daß die Magnesiumlegierung enthält:
Lithium in einem Anteil von zumindest 0,01 mas% und bis zu 7 mas%,
Aluminium in einem Anteil von zumindest 0,01 mas% und bis zu 16 mas%,
Yttrium in einem Anteil von zumindest 0,01 mas% bis zu 7 mas% sowie
Seltenerdmetalle ausgewählt aus einem Element mit der Ordnungszahl 57 bis 71 in einem Anteil von zumindest 0,01 mas% und bis zu 8 mas%.

Vorzugsweise ist die Magnesiumlegierung gemäß der Formel MgLi4Al4SE2 mas% (= 4 mas% Li + 4 mas% Al + 2 mas% SE + Rest Basiselement Mg) zusammengesetzt, wobei SE ein Seltenerdmetall ausgewählt aus einem Element mit der Ordnungszahl 57 bis 71 ist und wobei Yttrium in einem Anteil von 0,01 mas% bis 7 mas% enthalten ist. Alternativ kann die Magnesiumlegierung auch gemäß der Formel MgY4SE3Li2,4 mas% zusammengesetzt sein, wobei SE ebenfalls ein Seltenerdmetall ausgewählt aus einem Element mit der Ordnungszahl 57 bis 71 ist und wobei Aluminium in einem Anteil von 0,01 mas% bis 16 mas% enthalten ist. Das Seltenerdmetall, beispielsweise Cer, verbessert die mechanischen und korrosiven Eigenschaften, indem der Wasserstoff abgebunden wird und vermehrt Deckschichtkomponenten entstehen.

Die Magnesiumlegierung kann schmelzmetallurgisch, pulvermetallurgisch oder durch mechanisches Legieren zu einem Implantat ausgebildet werden oder durch Metallspritz-/Sintertechniken auf vorgefertigte Implantate appliziert werden. Die Werkstoffe können im guß- oder thermomechanisch behandelten Zustand als Implantat verwendet werden. Eine Erhöhung der mechanischen und/oder korrosiven Gebrauchseigenschaften tritt durch ein sequentielles Strangpressen, Homogenisieren und Auslagern auf. Die Implantate können darüber hinaus durch spanende oder formgebende Bearbeitung wie beispielsweise durch Drehen, Schmieden oder Stanzen hergestellt werden.

Die Erfindung nutzt die Seltenerdmetalle, die als Gruppe stark ähnelnde mechanische und korrosive Eigenschaften aufweisen und in die Legierung übertragen. Hier werden exemplarisch die Legierungskomponenten Cer, das als Repräsentant der Cer-basierten Mischmetalle ist, und Yttrium verwendet, da diese in der Gegenwart die höchste wirtschaftliche Verfügbarkeit aufweisen. Jedes andere Seltenerden-Element reagiert jedoch vergleichbar. Die Seltenerden bilden bei Korrosion Hydroxide, exemplarisch Ce(OH)₃, Aluminium bildet Spinelle wie MgAl₂O₄, Magnesium bildet eine MgO und Mg(OH)₂-Deckschicht aus. Durch den Zuschlag von Lithium werden diese Deckschichtkomponenten im angehobenen pH der Doppelschicht thermodynamisch stabiler und es werden weitere Deckschichtkomponenten wie z.B. Al(OH)₃ oder CeAlO₃ thermodynamisch erst möglich und stabil. Eine Anreicherung der Deckschicht durch mehr Komponenten führt zu einer Verdichtung, die zum einen die zugeigenspannungsbehaftete Mg(OH)₂-Deckschicht entspannt und durch die Verdichtung das Diffundieren von Mg reduziert. Durch weniger Mg in der Doppelschicht korrodiert das Implantat weniger, es entsteht weniger Wasserstoff. Durch weniger Wasserstoff wird das Implantat körperverträglicher und der pH-Wert bleibt auf höherem Niveau. So bleiben die z.T. pH-abhängigen Deckschichtkomponenten intakt und reduzieren die Korrosionsrate.

Die erfindungsgemäße Magnesiumlegierung kann in Form von chirurgischen Befestigungsdrähten unterschiedlicher Dicke, die auch aus Einzeldrähten geflochten sein können, von Schrauben insbesondere für die Hand- und Fußchirurgie sowie in der traumatologischen und orthopädischen Knochen- und Gelenkchirurgie insbesondere als Interferenzschrauben (Kreuzbandchirurgie) sowie als Naht- und Ankersystem zur Fixierung von Muskeln, Sehnen, Menisken, Gelenklippen (z.B. Acetabulum, Glenoid), Fascien, Periost und Knochen verwendet werden. Desweiteren kann die Magnesiumlegierung für Platten, Stifte, Knöpfe oder Cerclagen Verwendung finden.

In einer weiteren Anwendungsmöglichkeit können aus der erfindungsgemäßen Magnesiumlegierung Wund- oder Bruchnetze oder Wund- oder Bruchfolien hergestellt werden. Die Herstellung kann durch gegenseitiges Verbinden dünner Drähte oder durch Ausstanzen dünner Bleche erfolgen.

Darüber hinaus kann chirurgisches Nahtmaterial, insbesondere Wundklammern beispielsweise für Klammernahtgeräte, aus der Magnesiumlegierung bestehen.

Implantate mit einer Implantatbeschichtung mit der erfindungsgemäßen Magnesiumlegierung können insbesondere für Implantate mit Knochenkontakt Verwendung finden. Für die Aufbringung der Beschichtung können an sich bekannte Verfahren, z.B. thermisches Spritzen (Lichtbogen und Plasma), PVD (= physical vapor deposition), CVD (= chemical vapor deposition) oder Co-Strangpressen Anwendung finden.

Um die Verträglichkeit für den menschlichen oder tierischen Körper zu erhöhen, enthält die erfindungsgemäße Magnesiumlegierung kein Cadmium, d.h. sie ist cadmiumfrei.

Wenn die erfindungsgemäße Magnesiumlegierung für Prothesen oder Prothesenteile verwendet wird, ist der Vorteil gegeben, daß die Implantate nach dem Anwachsen des Knochens und dem dadurch gegebenen Erreichen der Sekundärstabilität resorbiert werden können, so daß der natürliche Kraftfluß innerhalb des Knochens nicht behindert wird.

Im folgenden erfolgt eine Beschreibung der Wirkungen der einzelnen Legierungskomponenten für die Legierungen vom Typ MgYSE und vom Typ MgLiAlSE, wobei SE, Seltenerden von Ce dominiert werden, so dass die Diskussion bzgl. SE an Hand von Ce exemplarisch geführt wird.

**Al, Aluminium**: Al-Zuschläge wirken sowohl gegen Korrosion in Freibewitterung als auch in Elektrolyten. Bei atmosphärischer Komplexbelastung weist Al-legiertes Mg im Vergleich zu Mg-Mn oder Mg-Fe geringere Deckschichtstärken auf. Geringere Oxidationsraten können mit vergleichsweise dichteren Deckschichten und somit einem gesteigertem Korrosionswiderstand einhergehen. Aufgrund der hohen Löslichkeit von 11,8 at% lässt sich das Gefüge über die Erstarrungsgeschwindigkeit stark modifizieren. Durch hohe Abkühlraten werden homogene Gefüge mit erhöhtem Korrosionswiderstand durch reduzierte Seigerung, Kornfeinung und weniger Lokalelemente erzeugt, bei niedrigen Abkühlungsraten entsteht ein heterogenes, gröber ausscheidungsdurchsetztes Gefüge. Im Allgemeinen sind jedoch heterogene Gefüge und somit Mikrolokalelemente zu vermeiden. In Mg(OH)₂-gesättigter 3 % NaCl-Lösung werden auf MgA13,5 mas% bzw. MgA110 mas% neben Mg(OH)₂, 3Mg(CO)₃·Mg(OH)₂·3H₂O auch Cl⁻, CO₃²⁻, O²⁻ und OH⁻ auch Al³⁺-Ionen detektiert, wobei die Al³⁺-IonenKonzentration mit der Korrosionszeit ansteigt. Al³⁺-Ionen verstärken die Deckschichtbildung nicht nur über Bildung des Spinells MgAl₂O₄ Magnesiumaluminat, sondern auch weil das dreiwertige Kation die o.g. Anionen zwecks Ladungsausgleich in der Deckschicht bindet. Dabei fungieren die Alreichen Ausscheidungen aufgrund ihres höheren Korrosionswiderstandes als Korrosionsbarrieren, weil die aluminiumreichen Oberflächenbereiche Mischoxide spenden. Eine MgAl9Zn1 (AZ91) weist in 5 % NaCl-Lösung im Gusszustand einen um Faktor 3 geringeren Korrosionswiderstand als das homogenisierte, gleichmäßig angereicherte Gefüge auf. Durch Auslagern werden eutektische Mg-Al-Ausscheidungen gebildet und der Korrosionswiderstand verdoppelt. Mit steigendem Al-Gehalt nimmt die Deckschichtstärke ab, da durch die Anreicherung mit Al-Kationen die Mg-Auflösung, mit der die deckschichtbildende Mg(OH)₂-Bildung einhergeht, reduziert wird. Es ist davon auszugehen, dass sich ab 4 mas% Al die Struktur und Stabilität des korrosionsschützenden Al₂O₃ nicht mehr ändert, dieses wird in MgO als MgO·Al₂O₃ integriert. MgO·Al₂O₃ ist jedoch nicht dem stöchiometrisch identischen MgAl₂O₄ gleichzusetzen: MgO·Al₂O₃-Elementarzelle: (B1·(D5₁, D5₆, H1₁)) ≠ MgAl₂O₄-Elementarzelle (H1₁). Mit steigendem Al-Gehalt der Mg-Legierung nimmt der Al₂O₃-Anteil in der Deckschicht und somit der Korrosionswiderstand zu. Durch Al-Zuschläge wird die intermetallische Korrosion reduziert Die korrosionsfördernde Affinität von Al zu Fe, die den Fe-Grenzwert absenkt und zur Bildung von kathodischem Fe₃Al mit E°_{5-NaCl} = -0,498 mV führt, kann durch Mn-Zuschläge, unabhängig von Sand- oder Druckguss kompensiert werden. Hohe Al-Gehalte bewirken eine Verschiebung der Maximalgrenzwerte für AZ91 (MgAl9Zn1) aus Druckguss für Fe auf 50 ppm, Ni auf 15 ppm und Cu auf 300 ppm. Allgemein kann die wichtigste Legierungskomponente des Magnesiums als korrosionsschützend in Gehalten von 1...9 mas% angesehen werden.

**Li, Lithium**: Die Entwicklung des Systems Mg-Li reicht bis 1910 zurück. In der zweiten Periode der Magnesiumentwicklung werden hochlithiumhaltige MgLi14 mas%-Basissysteme (MgLi38 at%) genutzt. Diese zeichnen sich durch eine Dichte von 1,4 g/cm³ und aufgrund der bei 30 at% kubisch raumzentrierten Gefügestruktur durch ein hohes Umformvermögen aus, was zur Herstellung von Blechen z.B. der Legierung MgLi12Al1 mas% (LA141) führt. Dieser superleichte Werkstoff wird nicht ungeschützt eingesetzt und das Korrosionsverhalten ist aufgrund der Legierungskomponente Li chemisch und elektrochemisch unbefriedigend und wird insgesamt als schlecht bewertet wird. Proben dieses Werkstoffes LAE141 laufen im Gusszustand bereits nach kurzer Zeit von vier Wochen dunkel an und zeigen nach rund einem halben Jahr das erste Chipping-Off (Abplatzen von plattenförmigen Segmenten des Volumens). Jedoch verringern Li-Zuschläge bis 10 mas% den Korrosionsangriff in 5 % NaCl-Lösung. In kochendem Wasser oder in Wasserdampf ist der Korrosionswiderstand umgekehrt proportional zum Li-Gehalt. Die Luftfeuchtigkeit spielt eine wesentliche Rolle bei der Korrosion von MgLi14 mas%, deren Korrosionswiderstand sich aber hier durch 1 mas% Al steigern lässt. Für Freibewitterung in urbanem, kontinentalem Seeklima weist MgLi40Al3Zn0,3 at%, homogenisiert mit (400°C/30min/Öl), dem geringen Korrosionsangriff auf, der einer AM20 recht nahe kommt: Die Riefen der spanenden Bearbeitung sind nach drei Monaten noch sichtbar, die Oberfläche glänzt schwach und schwarz. Nach 12 Monaten ist die Oberfläche der homogenisierten MgLi40Al3Zn0,3 at% zerrüttet. Für Li- und höher Al-haltige Mg-Werkstoffe ist die um 700 mV edlere AlLi-Phase korrosionskritisch, deren Volumenanteil durch das Homogenisieren reduziert wird. In synthetischem Meerwasser zeigt MgLi40Ca0,8 im Gusszustand die geringsten elektrochemischen Korrosionsraten. Chemisch und elektrochemisch alkalisierendes Li verschiebt den pH-Wert in der Doppelschicht in den Stabilitätsbereich von Mg(OH)₂ auf pH > 11,5. Die Mg(OH)₂₋Deckschicht wird in Demonstratorsystemen auf MgLi40 at%-Basis um die Legierungskomponenten Al, Zn oder Ca erweitert. Neben der grundsätzlichen Steigerung des Korrosionswiderstandes durch Mischoxide können Systeme MgLi40Al3Zn1 at% oder MgLi40Ca0,1 at% in 0,01 M H₂SO₄₋Lösung bzw. MgLi40Al3Zn1 at% in CO₂-begastem Leitungswasser einen gegenüber AZ91 (MgA19Zn1) gesteigerten Korrosionswiderstand aufweisen.

Die Legierungskomponente Li gilt als duktilitätssteigernd und korrosionsbeschleunigend. Insbesondere mit der Legierungskomponente Al im Zusammenspiel sind Systeme Mg-Li-Al durch die Bildung der stark korrosionsfördernden AlLi-Phase ungeschützt technisch nicht einsetzbar, ihre Verwendung bleibt auf Grund des aufwendigen Korrosionsschutzes auf militärische Bereich beschränkt.

**Seltenerden, SE, hier exemplarisch Ce**: Die Lanthanoide werden als Seltenerden, Seltenerdmetalle oder Mischmetalle bezeichnet. Die Seltenerden umfassen die Elemente der PSE-Ordnungszahlen 57...71 sowie Sc und Y, welche jedoch aus legierungstechnischer Sicht eine andere Stellung einnehmen und daher in ASTM-Nomenklatur und Diskussion unterschieden werden. Die Oxide werden Seltenerden oder Seltene Erden (SE, engl. RE bzw. HRE) genannt. Die Gruppenzusammenfassung erfolgt wegen stark ähnlicher chemischer und metallurgischer Eigenschaften, die in ähnlicher Weise auf Legierungen übertragen werden. Die freien Korrosionspotentiale der Seltenerden liegen in der Nähe von Mg, so dass ein Legieren von Mg nicht von vornherein als kritisch zu bewerten wäre. Cer-Zuschläge verringern jedoch die Korrosionsbeständigkeit. Für Mg-Al-Systeme erhöhen Ce-Zuschläge, die auf der Masseloberfläche als hellblauer bis violetter Glanz sichtbar sind, die Korrosionsbeständigkeit, unabhängig davon, ob Ce in einer Al-Ce-Ausscheidung, vgl. *Al*, oder homogenisiert (410°C/16h/Wasser) angeboten wird. Die dreilagige Deckschichttopographie des Systems Mg-Al wird durch Ce-Zuschlag Al-reicher und dehydriert, wodurch der Widerstand gegen den Durchtritt von Kationen steigt. Der Minimalgrenzwert für einen korrosionsschützenden Al-Gehalt wird durch Seltenerden, hier Ce abgesenkt. Ce ist die dominierende Komponente der Ce-basierten RE: 50 mas% Ce, 25 mas% La, 20 mas% Nd und 3 mas% Pr. Für Mg-Legierungen, die in Mg(OH)₂-gesättigter 5 % NaCl-Lösung korrodieren, wird der Korrosionsangriff insbesondere durch Nd abgesenkt. Nd zählt zur Gruppe der HRE, der Seltenerden mit höherer relativer Atommasse. Eine Mg-Y-Nd-Zr-Legierung hat dieselbe Korrosionsrate wie die technische Referenzlegierung MgAl9Zn1 (AZ91), weist jedoch eine geringere Lochtiefe auf. Dieses Phänomen wird mit einer SE-angereicherten Mg(OH)₂-Deckschicht erklärt. Auch Mg-Gd-Y-Zr zeigt einen guten Korrosionswiderstand. Y und Nd werden zum korrosionsschützenden Legieren empfohlen: MgDy10Nd3Zr0,4 mas% hat einer MgAl9Zn1 (AZ91D) vergleichbare Korrosionseigenschaften.

**Y, Yttrium**: Als Seltenerde weist Y ähnliche Korrosionseigenschaften wie SE auf: Bei einer maximalen Mischkristalllöslichkeit von 12,5 mas% für Y in Mg wird aber durch das Zulegieren von steigenden Y-Gehalten zu MgZn2 die Korrosionsrate in Flusswasser bis Y < 4 mas% auf ein Plateau doppelt so hoch wie MgZn2 angehoben, anschließend nimmt die Korrosionsrate stetig zu, für MgZn2Y12 ist die Korrosionsrate um Faktor 9 größer. Dies wird auf die steigende Durchsetzung des Mg-Zn-MK mit MgₓY_{z}-Phasen zurückgeführt.

### Kombination der Legierungssysteme

Die Übertragung des korrosionsschützenden Einflusses von Lithium in eine technische Legierung ist nicht nur unter korrosionsspezifischen Gesichtspunkten interessant. Der seigerungshemmende, korrosionswiderstands- und duktilitätssteigernde Li-Einfluss wird auf Mg-Al-SE-Systeme (AE) übertragen, da Aluminium zu Seltenen Erden eine höhere Affinität als zu Lithium aufweist. In anderen Fällen ist durch den Zuschlag von Li zu Mg-Al-Systemen die Bildung einer korrosionssteigernden AlLi-Phase zu erwarten. Diese würde nicht nur durch ihren kathodischen Charakter, sondern auch durch den Entzug von Al aus dem Mischkristall den Korrosionswiderstand senken. Andererseits kann Li nicht nur die natürliche Deckschicht Mg(OH)₂, sondern auch SE(OH)₃ und SEAlO₃ als weitere Deckschichtkomponenten durch die Dynamische Alkalisierung stabilisieren.

Die folgenden Ausführungen betreffen AE-Systeme mit gestuften Li-Gehalten: AE42, LAE242, LAE342, LAE442 und LAE542, wobei 12 at% Li aufgerundet 4 mas% entsprechen. Die Werkstoffe LAE452 und LAE472 werden auf Basis von Versuchen zum korrosionsschützenden Legieren 4 mas% Li und auf Basis der Kenntnisse von AM- und AZ-Systemen zur Gefügeausbildung in Abhängigkeit des Al-Gehaltes 7 mas% Al verwenden, wobei der Grenzwert für Al unter Berücksichtigung der Kornfeinung durch SE höher als die allgemein anzunehmenden 5 mas% liegt. Ein Maximierung des Al-Gehalts ist jedoch unter dem Aspekt der Deckschichtverdichtung mit Magnesiumaluminat anzustreben.

Mit Zunahme der Li-Komponente steigt die elektrochemische Korrosionsrate des AE-Systems bei gleichbleibendem Al-Gehalt von 4 mas% initial leicht an. Die makroskopischen Befunde der Korrosionsproben nach 200 h in synthetischem Meerwasser zeigen jedoch, dass die erhöhten Korrosionsraten von LAE542 und LAE452 durch einen erhöhten Angriff im Nahbereich der neutralen Faser des Gussbolzens und somit durch Entmischungen zu begründen sind.

Der metallische Glanz kann darauf zurückgeführt werden, dass gegenüber der Li-freien Variante, die als Deckschichtkomponenten Mg (OH)₂, MgAl₂O₄, Al(OH)₃ (letzteres nur bei mittleren pH-Werten) und Ce(OH)₃ aufweisen, als weitere CeAlO₃ durch die von der Li-Konzentration abhängige pH-Wertanhebung stabilisiert wird. Durch den angehobenen pH-Wert wird das Al(OH)₃ instabil, dies ist aber erwünscht, da das gesamte Aluminium in Aluminate übergeht. Durch die Steigerung des Al-Gehalts von 4 mas% über 5 mas% auf 7 mas% wird zum einen die Ausscheidungsdurchsetzung erwartungsgemäß drastisch erhöht sowie die Art der Ausscheidungen verändert. Zum anderen wird eine deutliche Verringerung der elektrochemischen Korrosionsrate erzielt. Das hochlegierte LAE472-System ergibt das korrosionsstabilste System.

Die thermomechanische Modifikation der LAE472 erfolgt aufeinander aufbauend als Gießen, Homogenisieren, Strangpressen und Auslagern. Im Gusszustand ist der Mg-Li-MK von großflächigen Kolonien der Al₁₁Ce₃-Phase durchsetzt. Das Homogenisieren soll nicht nur Ausscheidungen und Eigenspannungen durch das Kokillengießen reduzieren, sondern darüber hinaus definierte Homogenisierungs- und Auslagerungszustände einstellen. Das Homogenisieren wird für die Modellwerkstoffe mit (350 °C/4 h/Öl) durchgeführt, wobei die Halbzeuge in eine Wärmebehandlungsfolie eingeschlagen werden, die die Verdampfung reduziert, um das Ausdiffundieren von Lithium zu vermindern. Aufgrund der starken Ausscheidungsdurchsetzung kann das Gefüge einer LAE472 nicht vollkommen wie einer LAE442 homogenisiert und somit von Mikrolokalelementen bereinigt werden. Durch die Wärmebehandlung wird jedoch nicht ein Großteil der Ausscheidungen in die Matrix aufgesogen, sondern durch Koagulation eine deutliche Vergröberung und verstärkte Ausscheidungsdurchsetzung des Gefüges eingestellt. Entsprechend den Prinzipien des korrosionsschützenden Legierens sinkt somit der Korrosionswiderstand der homogenisierten LAE472 vs. LAE442 im Gusszustand.

Die thermomechanische Behandlung besteht aus zwei Schritten: einer halbstündige Vorwärmung bei 350°C und anschließendem Voll-Vorwärts-Fließpressen bei 300°C.

Durch das Strangpressen kommt es im Gefüge zu erneuten Ausscheidungen, die wiederum die mechanischen Eigenschaften und den Korrosionswiderstand beeinflussen. Durch ein erneutes Auslagern (180 °C/16 h/Öl) wird die Ausscheidungen gleichmäßig verteilt. Der Ausscheidungsanteil der globularen Al₂Ce- und lamellaren Al₄Ce-Phasen bleibt unverändert, das Korn wird jedoch feiner. Die Korrosionsrate der LAE472 ist im Gusszustand mit 0,04 mm/a in diesem Proben-Durchgang maximal, jedoch deutlich unter der Größenordnung des Ausgangswerkstoffes MgAl4SE2 mas% mit 0,2 mm/a. Die Korrosionsraten der wärmebehandelten Werkstoffe weisen auf ein weiteres Modell des korrosionsschützenden Legierens hin: Nicht die falsch homogenisierte und somit nicht lokalelementbefreite LAE72 weist die niedrigste Korrosionsrate auf, sondern die stranggepresste und durch Auslagerung mit feinen Lokalkathoden durchsetzte LAE472 mit 0,025 mm/a. Die definierte Durchsetzung der Matrix mit edleren Phasen, sogenannten lokalen Kathoden, einem weiteren Prinzip des korrosionsschützenden Legierens, führt zu einer Steigerung des Korrosionswiderstandes gegenüber dem Gußzustand. Allen LAE472-Systemen ist im elektrochemischen Korrosionsversuch im aggressiven Medium des synthetischen Meerwassers gemein, dass sie zum einen extrem geringe Korrosionsraten aufweisen, zum anderen kinetisch nicht stabilisiert sind: Die Korrosionsraten nehmen über der Zeit zu. Dieses Charakteristikum weisen die LAE-Systeme bis maximal 4 mas% Al für die Untersuchungsdauer von 200 h nicht auf. Diese Differenz erlaubt eine Klassifizierung in beschleunigt und "nur" korrodierende Implantatwerkstoffe, weil die hier dargestellten Korrosionsraten in vivo proportional größer ausfallen.

Die Übertragung der Deckschichtbildung unter Beteiligung von Seltenerden, hier Ce, führte auf die empirische Untersuchung des Korrosionswiderstandes von Mg-Y-SE- (WE)-Legierungen als Implantatwerkstoff in-vivo. Die Ergebnisse bestätigten auch hier den korrosionshemmenden Effekt von Ce, wobei diese Legierungen eine höherer Festigkeit und geringere Duktilität aufweisen. Auf Grund der unkritischen Konstitution der Zweistoffsysteme kann nach den Regeln der Metallurgie und Metallphysik abgeleitet werden, dass eine weitere Erhöhung des Seltenerden-Anteils den Korrosionswiderstand weiter erhöht.

## Patentansprüche

1. Medizinisches Implantat für den menschlichen und tierischen Körper, das zumindest teilweise aus einer Magnesiumlegierung besteht, die Anteile von Seltenerdmetallen und Lithium enthält, **dadurch gekennzeichnet**
**dass** die Magnesiumlegierung Lithium in einem Anteil von 0,01 mas% bis 7 mas%, Aluminium in einem Anteil von 0,01 mas% bis 16 mas%, Seltenerdmetalle ausgewählt aus einem Element mit der Ordnungszahl 57 bis 71 in einem Anteil von 0,01 mas% bis 8 mas% und Yttrium in einem Anteil von 0,01 mas% bis 7 mas% enthält.

2. Medizinisches Implantat nach Anspruch 1, **dadurch gekennzeichnet, dass** als Seltenerdmetalle Cer und/oder Neodym und/oder Praseodym Verwendung finden.

3. Medizinisches Implantat nach einem der Anspruch 1 oder 2, **dadurch gekennzeichnet, dass** die Magnesiumlegierung gemäß der Formel MgLi4A14SE2 mas% zusammengesetzt ist, wobei SE ein Seltenerdmetall ausgewählt aus einem Element mit der Ordnungszahl 57 bis 71 ist und wobei Yttrium in einem Anteil von 0,01 mas% bis 7 mas% enthalten ist.

4. Medizinisches Implantat nach einem der Ansprüche 1 bis 2, **dadurch gekennzeichnet, dass** die Magnesiumlegierung gemäß der Formel MgY4SE3Li2,4 mas% zusammengesetzt ist, wobei SE ein Seltenerdmetall ausgewählt aus einem Element mit der Ordnungszahl 57 bis 71 ist und wobei Aluminium in einem Anteil von 0,01 mas% bis 16mas% enthalten ist.

## Claims

1. Medical implant for the body of a person or an animal, made at least partially from a magnesium alloy containing portions of lithium and a rare earth metal, **characterized in that,** the magnesium alloy contains:
0,01 to 7 mass% lithium;
0,01 to 16 mass% aluminum;
a rare earth metal selected from an element having an atomic number of 57 to 71 in an amount of 0,01 to 8 mass%; and
0,01 to 7 mass% yttrium.

2. Medical implant according to claim 1, **characterized in that** the rare earth metal is cerium and/or neodymium and/or praseodymium.

3. Medical implant according to claim 1 or 2, **characterized in that** the magnesium alloy is composed according to the formula MgLi4A14RE2 mass%, wherein RE is a rare earth metal element having an atomic number of 57 through 71, and having 0,01 to 7 mass% yttrium.

4. Medical implant according to any one of the claims 1 to 2, **characterized in that** the magnesium alloy is composed according to the formula MgY4RE3Li2.4 mass%, wherein RE is a rare earth metal element of atomic number 57 through 71, and having 0,01 to 16 mass% aluminium.

## Revendications

1. Implant médical pour le corps humain ou animal, réalisé au moins partiellement en un alliage de magnésium contenant des proportions de lithium et d'un métal des terres rares, **caractérisé en ce que** l'alliage de magnésium contient
- du lithium dans une proportion allant de 0,01% à 7% en masse, de I#aluminium dans une proportion allant de 0,01 à 16 % en masse, des métaux des terres rares choisis parmi les éléments de numéro atomique allant de 57 à 71 dans une proportion allant de 0,01 à 8% en masse,
- de l'yttrium dans une proportion allant de 0,01 à 7% en masse.

2. Implant médical selon la revendication 1, **caractérisé en ce que** l'on utilise comme métal des terres rare du cérium (Ce) et/ou du néodyme et/ou du praséodyme.

3. Implant médical selon la revendication 1 ou 2, **caractérisé en ce que** l'alliage de magnésium est composé selon la formule de pourcentage massique MgLi4Al4SE2, SE étant un métal des terres rares choisi parmi les éléments de numéro atomique allant de 57 à 71, et l'yttrium étant compris dans une proportion allant de 0,01 à 71% en masse.

4. Implant médical selon l'une quelconque des revendications 1 à 2, **caractérisé en ce que** l'alliage de magnésium est composé selon la formule de pourcentage massique MgY4SE3Li2,4, SE étant un métal des terres rares choisi parmi un élément de numéro atomique allant de 57 à 71 et l'aluminium étant présent dans une proportion allant de 0,01 à 16% en masse.
